# EUROPÄISCHE PATENTSCHRIFT

(11) **EP 2 016 418 B1**
(45) Veröffentlichungstag und Bekanntmachung des Hinweises auf die Patenterteilung: **19.12.2012**
(21) Anmeldenummer: 07725231.0
(22) Anmeldetag: 15.05.2007
(51) Int. Cl.: G01N 33/68

(54) **IN VITRO VERFAHREN ZUR DIAGNOSE UND FRÜHDIAGNOSE VON NEURODEGENERATIVEN ERKRANGUNGEN**
IN VITRO PROCEDURE FOR DIAGNOSIS AND EARLY DIAGNOSIS OF NEURODEGENERATIVE DISEASES
PROCEDE IN VITRO POUR LE DIAGNOSTIC ET LE DIAGNOSTIC PRECOCE DE MALADIES NEURODEGENERATIVES

(30) Priorität: 17.05.2006 DE 102006023175
(43) Veröffentlichungstag der Anmeldung: 21.01.2009
(73) Patentinhaber: B.R.A.H.M.S GmbH, 16761 Hennigsdorf (DE)
(72) Erfinder: BERGMANN, Andreas, 13465 Berlin (DE); SPARWAßER, Andrea, 16761 Hennigsdorf (DE); HAMPEL, Harald, 80331 München (DE)
(74) Vertreter: Andrae, Steffen
(86) Internationale Anmeldenummer: PCT/EP2007/004314
(87) Internationale Veröffentlichungsnummer: WO 2007/131775

(56) Entgegenhaltungen:
- WO-A-2004/046181
- WO-A-2004/059293
- JP-A- 6 160 383
- ALBADALEJO M D ET AL: "[Plasmatic determination of natriuretic peptides in demented patients. Letter]" REVISTA DE NEUROLOGIA MAR 1997, Bd. 25, Nr. 139, März 1997 (1997-03), Seite 475, XP008082686 ISSN: 0210-0010 in der Anmeldung erwähnt
- MUDERS ET AL: "Evaluation of plasma natriuretic peptides as markers for left ventricular dysfunction" AMERICAN HEART JOURNAL, MOSBY- YEAR BOOK INC., ST. LOUIS, MO, US, Bd. 134, Nr. 3, September 1997 (1997-09), Seiten 442-449, XP005119735 ISSN: 0002-8703
- NILSSON KARIN ET AL: "Plasma homocysteine concentration and its relation to symptoms of vascular disease in psychogeriatric patients." DEMENTIA AND GERIATRIC COGNITIVE DISORDERS 2005, Bd. 20, Nr. 1, 2005, Seiten 35-41, XP008082685 ISSN: 1420-8008 in der Anmeldung erwähnt
- SJÖGREN M ET AL: "Increased intrathecal inflammatory activity in frontotemporal dementia: pathophysiological implications.", JOURNAL OF NEUROLOGY, NEUROSURGERY, AND PSYCHIATRY AUG 2004, vol. 75, no. 8, August 2004 (2004-08), pages 1107-1111, ISSN: 0022-3050
- RENTZOS M ET AL: "IL-15 is elevated in cerebrospinal fluid of patients with Alzheimer's disease and frontotemporal dementia.", JOURNAL OF GERIATRIC PSYCHIATRY AND NEUROLOGY JUN 2006, vol. 19, no. 2, June 2006 (2006-06), pages 114-117, ISSN: 0891-9887
- 'Atriales natriuretisches Peptid', [Online] Wikipedia Gefunden im Internet: <URL:http://de.wikipedia.org/wiki/Atriales_ natriuretisches_Peptid> [gefunden am 2010-11-11]

## Beschreibung

Die vorliegende Erfindung betrifft ein neues in vitro Verfahren für die Diagnose und insbesondere Frühdiagnose von neurodegenerativen Erkrankungen, insbesondere von Demenz-erkrankungen wie der Alzheimer Krankheit und deren Vorstufen.

Im Rahmen der vorliegenden Erfindung wird dabei der Begriff "Diagnose" als Oberbegriff für medizinische Bestimmungen gebraucht, denen je nach dem klinischen Zustand des Patienten, bei dem die Bestimmung durchgeführt wird, unterschiedliche Fragestellungen zugrunde liegen können und die der Erkennung und im vorliegenden Falle insbesondere auch der Früherkennung, der Bestimmung des Schweregrads und der Verlaufsbeurteilung, auch der therapiebegleitenden Verlaufsbeurteilung, und der Prognose des zukünftigen Verlaufs einer Erkrankung dienen. Dabei ist im vorliegenden Zusammenhang von besonderer Bedeutung, dass eine Diagnose auch eine Negativdiagnose sein kann, bei der aufgrund der Nichtfeststellbarkeit eines bestimmten krankheitstypischen Merkmals, z.B. der Nichtnachweisbarkeit eines mit der betreffenden Krankheit assoziierten Biomarkers in einer Blutprobe eines Patienten, das Vorliegen einer bestimmten Erkrankung unwahrscheinlich gemacht wird.

Für die Negativdiagnose sind auch Biomarker von großem Wert, die bei mehreren verschiedenen Krankheiten erhöht gefunden werden können und daher allein, für sich genommen, noch keine positive Diagnose einer spezielle Krankheit ermöglichen - obwohl sie in der Regel bei Hinzuziehung weiterer klinischer oder biochemischer Parameter auch für die Positivdiagnose entscheidend sein können.

Die Erkrankungen, um deren Diagnose es bei der vorliegenden Erfindung geht, sind sich eher langsam entwickelnde, chronische neurodegenerative Erkrankungen von nichtinfektiöser Ätiologie, insbesondere präsenile Demenz-Erkrankungen.

Als Demenz-Erkrankungen (Dementia) werden generell Krankheiten bezeichnet, für die ein gemeinsames Merkmal der Verlust erworbener intellektueller Fähigkeiten, v.a. des Gedächtnisses, und des normalen Persönlichkeitsniveaus als Folge von Hirnschädigungen ist. Demenzerkrankungen sind in der Regel sich relativ langsam entwickelnde Krankheiten von chronischem Charakter. Treten Demenzerscheinungen vor dem hohen Alter im mittleren Lebensalter auf, werden sie als präsenile Demenz-Erkrankungen bezeichnet, und bei diesen unterscheidet man auf der Basis der für sie typischen Symptome und hirnpathologischen Veränderungen insbesondere die folgenden Erkrankungen bzw. Gruppen von Erkrankungen:

Die **Alzheimer Demenz (AD)** (Alzheimer Krankheit; Morbus Alzheimer) ist die häufigste neurodegenerative Demenz-Erkrankung, macht 2/3 aller Demenzfälle aus und stellt auch das praktisch wichtigste Anwendungsgebiet für die vorliegende Erfindung dar. AD zeichnet sich durch drei wichtige, allerdings erst *post mortem* mit Sicherheit feststellbare pathologische Merkmale aus: die Bildung von Amyloid-Plaques und neurofibrillären Bündeln sowie den Verlust an Nervenzellen (Übersicht s. (1); Literaturangaben in der Beschreibung in Form von Zahlen beziehen sich auf die der Beschreibung folgende Literaturliste). Amyloid-Plaques bestehen aus extraneuronalen Aggregaten des Amyloid-ß-Proteins, während die Neurofibrillenbündel hauptsächlich Tau-Protein und Neurofilamente enthalten. Es wird vermutet, dass die Plaque - und Neurofibrillenbildung die Ursache für das Absterben von Nervenzellen ist.

Die wichtigsten Symptome von AD sind zunehmende Merkfähigkeits- und Denkstörungen bei relativ lang anhaltender Gemütsansprechbarkeit, wobei diese Symptome von weiteren weniger spezifischen Störungen begleitet werden, die die Abgrenzung der AD von anderen Demenzformen erschweren.

Beobachtungen an AD-Patienten und Patienten, die im Laufe ihrer langjährigen klinischen Beobachtung AD entwickelten, führten zur Formulierung von Kriterien für gegeneiander abgrenzbare Patientengruppen, die die ganze Breite von
(a) Personen ohne subjektive und objektive kognitive Störungen (die im Rahmen der vorliegenden Anmeldung die Kontrollgruppe repräsentieren), über
(b) Patienten, die über subjektiv empfundene kognitive Leistungseinbußen klagen, bei denen jedoch keine kognitiven Defizite festgestellt werden können (im Rahmen der vorliegenden Anmeldung ist das die Gruppe der "SKS"-Patienten, wobei "SKS" für "subjektive kognitive Störungen" steht), weiter über
(c) Patienten, bei denen leichte kognitive Störungen feststellbar sind und bei denen dann, wenn keine anderen demenzverursachenden Erkrankungen vorliegen, die Diagnose "möglicherweise AD" ("mgl AD") gestellt wird (im Rahmen der vorliegenden Anmeldung ist das die Gruppe "LKS mgl AD", wobei "LKS" für "leichte kognitive Störungen" steht) bis hin
(d) zur Gruppe der Patienten mit dem typischen Bild erheblicher kognitiver Störungen, die schleichend begonnen haben und langsam progredieren, für die dann, wenn andere Demenz-ursachen ausgeschlossen werden können, die Diagnose "wahrscheinlich AD" gestellt wird (im Rahmen der vorliegenden Anmeldung ist das die Gruppe "ws AD", wobei die Abkürzung für "wahrscheinlich Alzheimer" steht),
abdecken.

Bezüglich der Zuordnung von Patienten mit subjektiven und/- oder objektiven kognitiven Störungen zu verschiedenen Gruppen wird ergänzend verwiesen auf (2), (3), (4) und (5).

Die **Demenz mit Lewy-Körperchen** (dementia with lewy-bodies: DLB) ist nach der Alzheimer Demenz die zweithäufigste Ursache für eine demenzielle Erkrankung. Neuropathologisch ist die DLB durch das Auftreten von sogenannten Lewy-Körperchen im Hirnstamm und im Cortex charakterisiert. Diese LewyKörperchen bestehen überwiegend aus Aggregaten des präsynaptischen Proteins (α-Synuclein) und Ubiquitin. Die LewyBody-Pathologie kann in unterschiedlichem Ausmaß mit Alzheimer und Parkinson-typischen neuropathologischen Veränderungen assoziiert sein. So kommt es auch bei der DLB zur Bildung von beta-Amyloid und senilen Plaques, jedoch nicht zu Neurofibrillenbündeln (Übersicht s. (6)). Lewy-Körperchen sind auch im Gehirn von Patienten mit Morbus Parkinson vorhanden, wenn auch in einer unterschiedlichen Verteilung.

Kernsymptome von DLB sind eine progrediente kognitive Störung, Verwirrtheitsepisoden mit fluktuierender Aufmerksamkeits- und Bewusstseinslage, Parkinsonismus, häufige Stürze und Synkopen (anfallsartige, kurz dauernde Bewusstlosigkeit). Die Sensitivität und Spezifität der diagnostischen Kriterien zeigen durchgehend eine hohe Spezifität, aber zum Teil eine sehr niedrige Sensitivität. Das bedeutet, dass die DLB im klinischen Alltag häufig nicht diagnostiziert wird. Die **Frontotemporale Demenz (FTD)** wird auch als Pick'sche Krankheit bezeichnet und macht ca. 20% der präsenilen Demenzerkrankungen aus. FTD ist teilweise genetisch bedingt und zählt zu den sogenannten Tauopathien, die sich durch eine Über- oder Unterexpression eines Tauprotein-Subtyps bzw. durch die Expression eines mutierten Tauproteins auszeichnen. Neuropathologisch kommt es zu einer lokalen Atrophie des frontalen und/oder temporalen Cortex sowie der Substantia Nigra und der Basalganglien. Dies hat unterschiedlich ausgeprägte Sprachstörungen, eine Wesensänderung sowie Verhaltensauffälligkeiten zur Folge. Insgesamt ist die FTD mit einer Sensitivität von 93% bei einer Spezifität von nur 23% unterdiagnostiziert, wobei die AD die häufigste Fehldiagnose darstellt.

Unter dem Begriff **vaskuläre Demenz (vascular dementia; VAD)** werden Erkrankungen zusammengefasst, bei denen eine Demenz aufgrund von Durchblutungsstörungen im Gehirn ausgelöst wird. Es gibt unterschiedliche Typen der VAD, von denen die Multi-Infarkt-Demenz (MID) und die subcorticale VAD (auch als Binswanger'sche Erkrankung bezeichnet) die häufigsten Formen darstellen.

Bei der Binswanger'schen Erkrankung handelt es sich um eine langsam progrediente demenzielle Entwicklung, die pathologisch durch cerebrovasculäre Läsionen in der weißen Hirnsubstanz charakterisiert ist. Klinisch resultiert dies in Verhaltensauffälligkeiten wie Agitation, Reizbarkeit, Depression und Euphorie sowie einer leichten Gedächtnisstörung.

Die Multi-Infarkt-Demenz entsteht allmählich als Folge von mehreren kleinen Schlaganfällen, auch als transiente ischämische Attacken (TIA) bezeichnet, die zum Untergang von Hirngewebe im Cortex und/oder subcortikalen Arealen führten. Die Schlaganfälle können auch gänzlich unbemerkt geblieben sein, die Demenz ist in diesem Falle die erste spürbare Folge. Bei Vorliegen einer MID kommt es zur stufenweisen Abnahme kognitiver Fähigkeiten, verbunden mit schweren Depressionen, Stimmungsschwankungen und Epilepsie.

Eine Diagnose auf Demenz-Erkrankungen erfolgt heutzutage überwiegend auf der Basis neuropsychologischer Untersuchungen und der Beobachtung der Krankheitsentwicklung und ihres Verlaufs, unter Heranziehung von Ausschlusskriterien für bestimmte Demenzformen. Diese Untersuchungen liefern in sehr vielen Fällen mehrdeutige Ergebnisse, die die o.g. Zahlen für die unterdiagnostizierten Demenzformen oder unrichtig diagnostizierten Fälle erklären. Die krankheitstypischen Gehirnveränderungen können an lebenden Patienten naturgemäß nicht direkt festgestellt werden, und apparatemedizinische Untersuchungen der Gehirnfunktionen mittels z.B. Röntgen - oder Kernspin-Tomographie sind aufwändig und teuer.

Es wäre daher wünschenswert, die Erkennung und insbesondere Früherkennung von Demenzerkrankungen durch die Messung von aussagekräftigen Biomarkern, die mit Hilfe eines relativ einfachen Testverfahren z.B. in einer Blutprobe (Serumprobe, Plasmaprobe) eines Patienten bestimmt werden können, ergänzen und dadurch erheblich verbessern zu können.

Für die Alzheimer-Diagnostik veröffentlichten das Ronald und Nancy Reagan Institut der Alzheimer Association und die NIA-Working Group Richtlinien für die Kriterien, die an einen idealen Biomarker zur Detektion der AD gestellt werden (7). Folgende Kriterien sollen im Idealfall durch den Biomarker erfüllt werden:
1. Er sollte hirnspezifisch sein und ein fundamentales Merkmal der Neuropathologie dieser Erkrankungen detektieren.
2. Die diagnostische Sensitivität und die Spezifität von mindestens 80% sollte gegeben sein.
3. Die krankheitsspezifische veränderung des Biomarkers sollte sich in einem möglichst frühen Stadium der Erkrankung manifestieren, um mit geeigneten Therapiemaßnahmen beginnen zu können (8).

Bis jetzt gibt es jedoch keinen Biomarker, der im klinischen Alltag im Blut oder der cerebrospinalen Flüssigkeit mit ausreichender Sicherheit zur Früh- und Differentialdiagnose von AD herangezogen werden könnte und alle o.g. Kriterien erfüllt. Aktuell werden verschiedene potentielle Markerkandidaten untersucht, darunter Inflammationsmarker wie IL-6 und TNFα, Marker für oxidativen Stress wie 3-Nitrotyrosin, sowie Marker, die mit charakteristischen pathologischen Veränderungen der AD assoziiert sind, wie Amyloid ß, das einen Hauptbestandteil der Amyloid-Plaques darstellt, und das Tau-Protein, das einen wesentlichen Bestandteil der Neurofibrillenbündel darstellt (vgl. die Übersicht in (7); (10) ) .

Es besteht ein aktueller Bedarf nach ergänzenden, valide Laborbefunde liefernden Untersuchungsmethoden, die auf einer Bestimmung von als Biomarker für Demenzerkrankungen, insbesondere für die Alzheimer Demenz (AD), geeigneten Substanzen in Blut- bzw. Plasmaproben beruhen und bei Patienten, bei denen der Verdacht auf Vorliegen einer Demenzerkrankung, insbesondere von AD, besteht, unterstützend für eine frühe Positivdiagnose und/oder für eine negative Ausschlussdiagnose geeignet sind.

Die vorliegende Erfindung stellt eine solche Untersuchungsmethode in Form eines *in vitro* Verfahrens gemäβ auspruch 1 bereil.

Vorteilhafte bzw. bevorzugte Ausgestaltungen eines Verfahrens gemäß Anspruch 1 sind in den Unteransprüchen 2 bis 8 wiedergegeben.

Als "natriuretische Peptide oder deren aus einem gemeinsamen Propeptid gebildete Copeptide" sind insbesondere anzusehen die Peptide ANP (das "atrial-natriuretsche Peptid"), dessen Bestimmung in Form der Bestimmung des sog. NT-proANP (des N-terminalen pro-ANP, d.h. des N-terminalen Teilpeptids, das bei der Freisetzung von ANP aus dem gemeinsamen Vorläufer proANP gebildet wird) erfolgt, BNP (des sogenannten "brain-natriuretischen Peptids"), dessen Bestimmung ebenfalls in Form der Bestimmung des sog. NT-proBNP (des N-terminalen pro-BNP, d.h. des N-terminalen Teilpeptids, das bei der Freisetzung von BNP aus dem gemeinsamen Vorläufer proBNP gebildet wird), erfolgt, sowie das verwandte sog. CNP, das die kürzeste Peptidkette von den genannten natriuretischen Peptiden aufweist.

In Rahmen der vorliegenden Erfindung wird ANP als MR-proANP bestiment indem die Bestimmung von NT-proANP vorzugsweise mit Hilfe eines Immunoassays erfolgt, der Aminosäuresequenzen im midregionalen Bereich (MR) des proANP bzw. NT-proANP erkennt und der in näheren Einzelheiten beschrieben ist in WO 2004/046181 bzw. dem daraus hervorgegangenen Europäischen Patent EP 1 562 984 B1. Eine weitere Beschreibung dieses Immunoassays findet sich in (9).

Eine bevorzugte Variante des erfindungsgemäßen Verfahrens besteht darin, nicht nur ein natriuretisches Peptid allein zu bestimmen, sondern neben der Bestimmung von ANP, die vorzugsweise nach dem o.g. Bestimmungsverfahren erfolgt, auch noch BNP nach irgendeinem bekannten Verfahren zur direkten oder indirekten Bestimmung von BNP zu bestimmen und die Messergebnisse für die Bestimmung von ANP und BNP gemeinsam, üblicherweise unter gleichzeitiger Berücksichtigung der klinischen Befunde und anderer Testparameter für den jeweiligen Patienten, zu betrachten und zur Auswertung heran zu ziehen.

Der vorliegenden Erfindung liegen Überlegungen der Erfinder zugrunde, zur Verbesserung der Diagnostik von Demenz-Erkrankungen bei der Erkenntnis anzusetzen, dass die bekannten, eingangs näher erläuterten Formen der präsenilen Demenz auch - in unterschiedlichem Ausmaß - von entzündlichen (inflammatorischen) Prozessen und Endothelschäden begleitet sind, die als wesentlich für die Entwicklung, die Symptome und den Verlauf der Demenzerkrankungen angesehen werden, weshalb neurodegenerative Erkrankungen auch als neuroinflammatorische Erkrankungen angesehen werden können.

So ist Morbus Alzheimer u.a. durch das Auftreten chronischer lokaler Entzündungsreaktionen im Gehirn unter Beteiligung von verschiedenen inflammatorischen Proteinen wie Komplement-Faktoren, Akutphasen-Proteinen und proinflammatorischen Cytokinen gekennzeichnet (10).

Inflammatorische Prozesse spielen auch eine Rolle bei der Entstehung von vaskulären Demenzen (VAD). Die Level von TNFα, TGFß, IL-6 und GM-CSF (Granulozyten-Makrophagen-stimulierender Faktor) sind bei Patienten mit VAD deutlich erhöht (11, 12).

Auch bei DLB scheinen inflammatorische Prozesse eine Rolle zu spielen. So ist die Zahl der aktivierten Mikroglia-Zellen im Gehirn von Patienten mit DLB erhöht, und proinflammatorische Cytokine wie TNFα werden in bestimmten Hirnregionen wie der Amygdala und dem Hippocampus überexprimiert.

Für das Auftreten inflammatorischer Reaktionen im Gehirn von FTD-Patienten gibt es dagegen nur vereinzelte Hinweise.

Ausgehend (i) von der Hypothese, dass die mit Demenzerkrankungen verbundenen neuroinflammatorischen Prozesse zu Durchblutungsstörungen, insbesondere auch zu Mikrozirkulationsstörungen des Gehirns führen, und (ii) dass insoweit eine Ähnlichkeit mit cardiovasculären Erkrankungen besteht, die mit Durchblutungsstörungen bzw. Störungen der Mikrozirkulation (13) des Herzgewebes verbunden sind, sowie (iii) von analytischen Befunden, die zeigen, dass bei solchen cardiovasculären Erkrankungen eine erhöhten Bildung u.a. der natriuretischen Peptide ANP und/oder BNP feststellbar ist, sowie schließlich (iv) unter Nutzung der verbesserten analytischen Möglichkeiten zur Bestimmung des natriuretischen Peptids in zuverlässiger und in klinisch valider Form mit Hilfe des o.g. neuartigen Immunoassays der Anmelderin zur Bestimmung des mittleren Abschnitts von proANP (MR-proANP) gemäß (9), untersuchten die Erfinder die Frage, ob sich auch bei Patienten mit kognitiven Störungen unterschiedlichen Ausmaßes, die ansonsten an keiner bekannten, mit einer erhöhten Produktion natriuretischer Peptide verbundenen Erkrankung litten, im Plasma Hinweise auf erhöhte Konzentrationen von ANP finden lassen.

Soweit in der Literatur Versuche der Messung natriuretischer Peptide bei Personen beschrieben wurden, die demenzartige Symptome zeigten, war keine signifikante Korrelation festgestellt worden (14, 15).

Die nachfolgend im experimentellen Teil beschriebenen Messergebnisse in EDTA-Plasmaproben von augenscheinlich 106 gesunden Normalpersonen (symptomfreien Kontrollen) und 196 Patienten mit leichten bis schweren kognitiven Störungen gemäß den eingangs beschrieben Gruppen (b) bis (d) ergab erstmals eine klare, diagnostisch signifikante Korrelation zwischen den für MR-proANP gefundenen Konzentrationen und der Schwere der Demenzsymptome in Form kognitiver Störungen, wobei die gemessenen Konzentrationen in signifikanter Weise mit der Schwere der Störungen und damit AD-Vorstufen korrelierten und damit zur Unterscheidung der verschiedenen Patientengruppen beitrug.

Obwohl die Untersuchungen bisher auf Plasmaproben von Patienten beschränkt waren, die Anzeichen von Vorstufen von AD zeigten bzw. für die die Diagnose "wahrscheinlich Alzheimer" gestellt worden war, gehen die Erfinder davon aus, dass - möglicherweise mit unterschiedlichen typischen Konzentrationsbereichen - auch bei anderen neuroinflammatorischen Demenzformen, insbesondere bei vaskulärer Demenz (VAD) und Demenz mit Lewy-Körperchen (DLB), charakteristische Erhöhungen der MR-proANP Konzentrationen in Patientenplasmen feststellbar sein könnten.

Das für die im experimentellen Teil beschriebenen Messungen eingesetzte Bestimmungsverfahren für MR-proANP in Patientenplasmen erfolgte mit dem o.g. nichtkompetitiven immunoluminometrischen Sandwichassay (B.R.A.H.M.S SERISTRA®), der in näheren Einzelheiten in der WO 2004/046181 der Anmelderin bzw. in (9) beschrieben wird. Auf die allgemeinen Ausführungen zur Problematik der ANP-Bestimmung in Patientenproben und die Erläuterungen zur Assaydurchführung in den genannten Publikationen wird zur Ergänzung der Ausführungen in der vorliegenden Anmeldung ausdrücklich Bezug genommen.

Nachfolgend wird die Erfindung anhand von Messergebnissen und einer Figur noch näher erläutert.

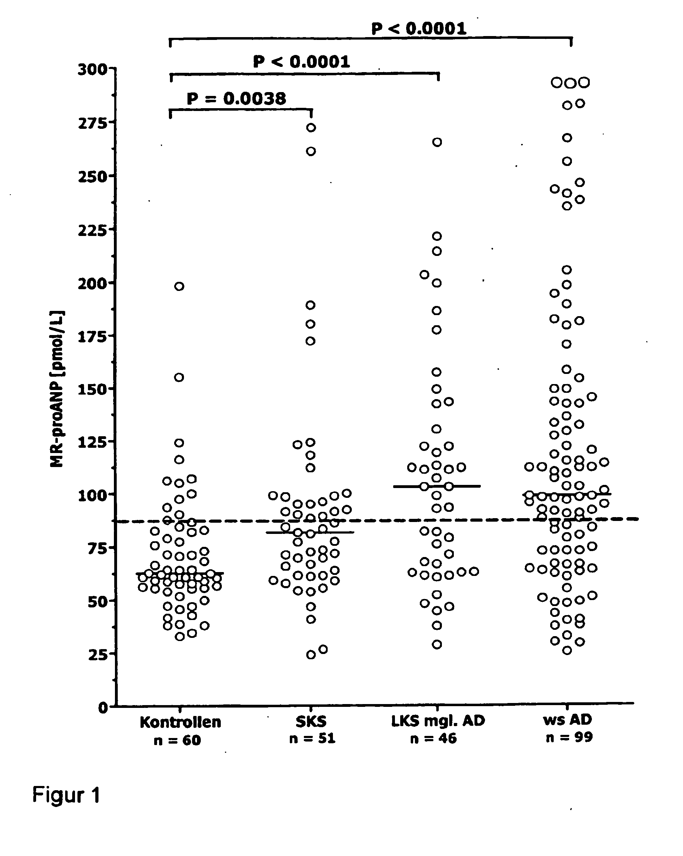

- Figur 1: zeigt die Ergebnisse der Messung der MR-proANP-Konzentrationen in EDTA-Plasmen von 106 gesunden Kontrollpersonen, und von 196 Patienten mit kognitiven Störungen verschiedener Schwere, die den o.g. Gruppen (b), (c) und (d), d.h. den Gruppen "SKS" (50 Patienten), "LKS mgl AD" (46 Patienten) und "ws AD" (100 Patienten) entsprachen.

### Experimenteller Teil

### Assaybeschreibung

Die Messung von MR-proANP im Plasma erfolgte mit einem immunoluminometrischen Sandwichassay im wesentlichen so wie im experimentellen Teil der o.g. WO 2004/046181 bzw. in (9) beschrieben wird.

Und zwar wurden 10 µl Probe/Kalibrator und 200 µl Tracer (markierter erster Antikörper) in die mit dem zweiten Antikörper beschichteten Röhrchen gegeben und unter Mischen (170-300 U/min) zwei Stunden bei Raumtemperatur (18-24°C) inkubiert. Dann wurde die flüssige Phase dekantiert, und die Röhrchen wurden viermal mit 1ml LUMItest Waschlösung (B.R.A.H.M.S Aktiengesellschaft, Hennigsdorf, Deutschland) gewaschen. Danach wurde die gebundene Chemilumineszenz für 1 s pro Röhrchen mit einem LB952T-Luminometer (Berthold, Wildbad, Deutschland) gemessen.

### Messung von MR-proANP im Plasma von gesunden Kontrollen und Patienten mit kognitiven Störungen verschiedenen Schweregrads

Zur Ermittlung eines Bezugswerts für die Konzentration des MR-proANP wurde eine Messung in EDTA-Plasmen von 106 symptomfreien Kontrollpersonen durchgeführt, die weder Symptome kognitiver Störungen zeigten noch an irgendeiner anderen erkennbaren Erkrankung (cardiovasculäre Erkrankungen; schwere Infektion oder Entzündung) litten, für die bekannt ist, dass bei ihr die natriuretischen Peptide ANP bzw. BNP erhöht gemessen werden können. Für die Kontrollgruppe wurde ein Mittelwert (Median) für die gemessene MR-proANP-Konzentration von 63,45 pmol/L ermittelt.

Als Patientenkollektiv dienten Patienten mit Demenzerscheinungen in Form kognitiver Störungen verschiedener Schwere, aufgrund derer eine Zuordnung der einzelnen Patienten zu einer der o.g. Gruppen (b), (c) bzw (d) erfolgt war.

Die gemessenen MR-proANP-Konzentrationen im Plasma von gesunden Kontrollen und Patienten mit kognitiven Störungen sind in Figur 1 gezeigt.

Die ermittelten Zahlenwerte in Form der sog. Mediane für die verschiedenen Patientengruppen sowie die aus den Messdaten unter Verwendung des angegebenen Werts für den Cut-off von 87,0 pmol/L errechneten Spezifitäten und Sensitivitäten für die verschiedenen Patientengruppen waren wie folgt:

| | Median Kontrollen | Median SKS | Median LKS mgl AD | Median ws AD |
|---|---|---|---|---|
| MR-proANP (Angaben in pmol/L) | 62,6 | 81,5 | 103,0 | 98,8 |

| | Cut off (pmol/L) | Spezifität | Sensitivität | Sensitivität | Sensitivität |
|---|---|---|---|---|---|
| | | (%) | SKS (%) | LKS mgl AD (%) | ws AD (%) |
| MM-proANP | 87,0 | 81,7 | 43,1 | 58,7 | 64.7 |

Die MR-proANP-Konzentrationen, erkennbar an den Werten für die Mediane der verschiedenen Patientengruppen, steigen mit der Schwere der Symptome in der Richtung:
Kontrollen < SKS < LKS mgl AD = ws AD klar an.

Vorläufige orientierende Bestimmungen der Konzentrationen von BNP (unter Verwendung eines kommerziellen NT-proBNP-Kits der Fa. Roche Diagnostics) bei Patienten der gleichen Patientengruppen ergab bei einer Spezifität von 80,2 % Sensitivitäten für die SKS-Gruppe von 38,0 %, für die Gruppe "LKS mgl AD" von 58,0 % und für die Gruppe "ws AD" von 61,0 %.

Die Messung von BNP bestätigt somit im wesentlichen den Befund der Messungen von ANP (als MR-proANP). Da es bekannt ist, dass in einer solchen Situation die Signifikanz von Bestimmungen in der Regel verbessert wird, wenn man mehr als einen Parameter misst und die Ergebnisse beider Messungen für die Auswertung gemeinsame betrachtet und/oder in geeigneter Weise rechnerisch kombiniert, liegt die gemeinsame Bestimmung von ANP sowie BNP und/oder ggf. CNP ausdrücklich im Bereich der vorliegenden Erfindung.

Obwohl eine erhöhte Freisetzung natriuretischer Peptide, z.B. von ANP, gemessen als MR-proANP Konzentration in Plasma, auch bei anderen Erkrankungen (Sepsis; cardiovaskuläre Erkrankungen/Fierzinsuffizienz; diese sind klinisch jedoch in der Regel einfach von Demenzerkrankungen abgrenzbar) messbar ist und natriuretische Peptide daher keine gehirnspezifischen Parameter darstellen, eignet sich die ANP-Bestimmung, ggf. in Kombination mit einer BNP- und/oder CNP-Bestimmung, aufgrund der hohen Spezifität und den klar voneinander unterscheidbaren Sensitivitäten sehr gut für Zwecke der unterstützenden AD-Frühdiagnostik.

### Literatur

1. SELKOE D. J. (2001). Alzheimer's disease: genes, proteins, and therapy. Physiological Reviews 81: 741-766
2. Boetsch T., Stübner S. Auer S., Klinisches Bild, Verlauf und Prognose, Kapitel 5 in: Hampel, Padberg, Möller (Hrsg.), Alzheimer Demenz - Klinische Verläufe, diagnostische Möglichkeiten, moderne Therapiestrategien; WVG mbH Stuttgart 2003
3. Boetsch T., Operationalisierte Demenzdiagnostik, Kapitel 6.1 in: Hampel, Padberg, Möller (Hrsg.), Alzheimer Demenz - Klinische Verläufe, diagnostische Möglichkeiten, moderne Therapiestrategien; WVG mbH Stuttgart 2003
4. Reisberg B., Ferris S.H., de Leon M.J., Crook T., 1982, The global deterioration scale for assessment of primary degenerative dementia, Am J Psychiatry 139:1136-1139
5. McKhann G., Drachmann D., Folstein M., Katzman R., Price D., Stadlan E.M. 1984, Clinical diagnosis of Alzheimer's disease: Report of the NINCDS-ARDA work grop under the auspices of departement of health services task force on Alzheimer's disease, Neurology 24: 939-944
6. MCKEITH I. G. (2002). Dementia with lewy bodies. British Journal of Psychiatry 180: 144-147
7. FRANK R. A., GALASKO D., HAMPEL H., HARDY J., DE LEON M. J., MEHTA P. D., ROGERS J., SIEMERS E., TROJANOWSKI J. Q. (2003). Biological markers for therapeutic trials in Alzheimer's disease. Proceedings of the biological markers working group; NIA initiative on neuroimaging in Alzheimer's disease. Neurobiology of Aging 24: 521-536
8. GROWDON J. H., SELKOE D. J., ROSES A., TROJANOWSKI J. Q., DAVIES P., APPEL S. et al. [Working Group Advisory Committee] . (1998). Consensus report of the Working Group on Biological Markers of Alzheimer's Disease. [Ronald und Nancy Reagan Institute of the Alzheimer's Association and National Institute on Aging Working Group on Biological Biomarkers of Alzheimer's Disease]. Neurobiology of Aging 19: 109-116
9. Nils G. Morgenthaler, Joachim Struck, Barbara Thomas, Andreas Bergmann, "Immunoluminometric Assay for the Midregion of Pro-Atrial Natriuretic Peptide in Human Plasma", Clinical Chemistry 50, No. 1, 2004, S. 234-236.
10. TEUNISSEN C. E., DE VENTE J., STEINBUSCH H. W. M., DE BRUIJN C. (2002). Biochemical markers related to Alzheimer's dementia in serum and cerebrospinal fluid. Neurobiology of Aging 23:485-508
11. TARKOWSKI E. (2002). Cytokines in dementias. Current Drug Targets - Inflammation and Allergy 1: 193-200
12. TARKOWSKI E., LILJEROTH A. M., MINTHON L., TARKOWSKI A., WALLIN A., BLENNOW K. (2003). Cerebral pattern of pro- and anti-inflammatory cytokines in dementias. Brain Research Bulletin 61: 255-260
13. CHU D.Q., SMITH D.M., BRAIN S.D.(2001). Studies of the microvascular effects of adrenomedullin and related peptides. Peptides 22:1881-1886
14. Karin Nilsson, Lars Gustavson, Björn Hultberg, Plasma Homocystein Concentration and Its Relation to Symptoms of Vascular Disease in Psychogeriatric Patients, Dement Geriatr Cogn Disord 2005; 20:35-41
15. M.D. Albadalejo, M. Antem, I. Pastor, C. Ruiz, R. Gonzalez-Aniorte, M. Asensio, Determinacion plasmatica de peptidos natriureticosen dementes, Rev Neurol 1997; 25 (139)

## Patentansprüche

1. *In vitro* Verfahren zur Erkennung und Früherkennung, zur Bestimmung des Schweregrads und zur Verlaufsbeurteilung und Prognose von Alzheimer Demenz (AD), Demenz mit Lewy-Körperchen (DLB), frontotemporaler Demenz (FTD) oder verschiedenen Formen der vaskulären Demenz (VD), bei dem man im Blut eines Patienten, der an subjektiven oder objektiv nachweisbaren kognitiven Störungen leidet, mit Hilfe eines immundiagnostischen Bestimmungsverfahrens, einen mittleren Abschnitt des pro-atrialnatriuretischen Peptids (mr-proANP) detektiert und bei dem man auf der Basis der gemessenen Konzentration Schlüsse hinsichtlich des Vorliegens einer neurodegenerativen Erkrankung oder einer für diese typischen Frühform davon oder hinsichtlich des Verlaufs der Erkrankung und/oder des Erfolgs der Bemühungen zu ihrer Abmilderung oder Verhinderung zieht.

2. Verfahren nach Anspruch 1, **dadurch gekennzeichnet, dass** man neben mr-proANP zusätzlich BNP und/oder CNP oder ein zugehöriges, aus einem entsprechenden Propeptid gebildetes BNP- oder CNP-Copeptid bestimmt.

3. Verfahren nach einem der Ansprüche 1 oder 2, **dadurch gekennzeichnet, dass** das immundiagnostische Bestimmungsverfahren ein Immunoassay vom Sandwichtyp ist.

4. Verfahren nach Anspruch 1, **dadurch gekennzeichnet, dass** es im Rahmen der Alzheimer-Diagnostik zur Erkennung von Frühformen der Alzheimer Demenz (AD) durchgeführt wird.

5. Verfahren nach einem der Ansprüche 1 bis 4, **dadurch gekennzeichnet, dass** es im Rahmen einer Multiparameter-Bestimmung durchgeführt wird, bei der gleichzeitig mindestens ein weiterer für das jeweilige Krankheitsbild aussagekräftiger biochemischer oder physiologischer Parameter bestimmt wird und bei der ein Messergebnis in Form eines Satzes von mindestens zwei Messgrößen gewonnen wird, der zur Feindiagnostik der neurodegenerativen Erkrankung ausgewertet wird.

6. Verfahren nach Anspruch 5, **dadurch gekennzeichnet, dass** im Rahmen der Multiparameter-Bestimmung neben der Bestimmung von mr-proANP wenigstens ein weiterer biochemischer Parameter bestimmt wird, der aus den Gruppen der Entzündungsmediatoren, Komplementkomponenten, Cytokine, Chemokine, der Blutkoagulanzien und fibrinolytischen Faktoren, Akutphasenproteine und radikalischen Verbindungen ausgewählt ist.

7. Verfahren nach Anspruch 5 oder 6, **dadurch gekennzeichnet, dass** die Multiparameter-Bestimmung als Simultanbestimmung mittels einer Chiptechnologie-Messvorrichtung oder einer immunchromatographischen Messvorrichtung erfolgt.

8. Verfahren nach Anspruch einem der Ansprüche 5 bis 7, **dadurch gekennzeichnet, dass** die Auswertung des komplexen Messergebnisses der Multiparameter-Bestimmung mit Hilfe eines Computerprogramms erfolgt.

## Claims

1. *In vitro* method for the detection and early detection, for the determination of the severity and for the assessment of the course and prognosis of Alzheimer's disease (AD), dementia with Lewy bodies (DLB), frontotemporal dementia (FTD) and various forms of vascular dementia (VD), in which in the blood of a patient who is suffering from subjective or objectively detectable cognitive disturbances a midregion of pro-atrial natriuretic peptide (mr-proANP) is detected with the aid of an immunodiagnostic assay method, and in which conclusions regarding the presence of a neurodegenerative disease or of an early form thereof typical of said disease or regarding the course of the disease and/or the success of the efforts for its alleviation or prevention are drawn on the basis of the detected concentration.

2. Method according to Claim 1, **characterized in that** in addition to mr-proANP also BNP and/or CNP or a related BNP- or CNP-copeptide formed from the corresponding propeptide are determined.

3. Method according to either of Claims 1 or 2, **characterized in that** the immunodiagnostic assay method is an immunoassay of the sandwich type.

4. Method according to Claim 1, **characterized in that** it is carried out as part of Alzheimer's diagnosis for the detection of early forms of Alzheimer's disease (AD).

5. Method according to any of Claims 1 to 4, **characterized in that** it is carried out as part of a multiparameter determination in which simultaneously at least one further biochemical or physiological parameter informative for the respective clinical picture is determined and in which a measured result is obtained in the form of a set of at least two measured variables, which is evaluated for the fine diagnosis of the neurodegenerative disease.

6. Method according to Claim 5, **characterized in that** as part of the multiparameter determination, in addition to the determination of mr-proANP, at least one further biochemical parameter is determined which is selected from the group consisting of inflammation mediators, complement components, cytokines, chemokines, blood coagulants and fibrinolytic factors, acute phase proteins and radical compounds.

7. Method according to Claim 5 or 6, **characterized in that** the multiparameter determination is effected as a simultaneous determination by means of a chip technology measuring apparatus or an immunochromatographic measuring device.

8. Method according to any of claims 5 to 7, **characterized in that** the evaluation of the complex measured result of the multiparameter determination is effected with the aid of a computer program.

## Revendications

1. Procédé *in vitro* de détection et de détection précoce, pour la détermination de la gravité et pour l'évaluation de l'évolution et du pronostic de la maladie d'Alzheimer (AD), de la démence avec présence de corps de Lewy (DLB), de la démence fronto-temporale (FTD) et de diverses formes de démence vasculaire (VD), dans lequel, dans le sang d'un patient qui souffre de perturbations cognitives détectables de manière subjective ou objective, une région médiane du peptide natriurétique pro-atrial (mr-proANP) est détectée au moyen d'une méthode d'analyse d'immunodiagnostic, et dans lequel les conclusions concernant la présence d'une maladie neuro-dégénérative ou d'une forme précoce de celle-ci caractéristique de ladite maladie ou concernant l'évolution de la maladie et/ou le succès des tentatives de soulagement ou de prévention de celle-ci sont tirées sur la base de la concentration détectée.

2. Procédé suivant la revendication 1, **caractérisé en ce que**, en plus de mr-proANP, le BNP et/ou le CNP, ou un BNP- ou CNP-copeptide apparenté formé à partir du propeptide correspondant, sont également déterminés.

3. Procédé suivant la revendication 1 ou la revendication 2, **caractérisé en ce que** la méthode d'analyse d'immunodiagnostic est une analyse immunologique du type en sandwich.

4. Procédé suivant la revendication 1, **caractérisé en ce qu'**il est mis en oeuvre en tant qu'une partie du diagnostic de la maladie d'Alzheimer pour la détection de formes précoces de la maladie d'Alzheimer (AD).

5. Procédé suivant l'une quelconque des revendications 1 à 4, **caractérisé en ce qu'**il est mis en oeuvre en tant qu'une partie d'une détermination multiparamétrique dans laquelle, simultanément, au moins un paramètre biochimique ou physiologique supplémentaire informatif pour le tableau clinique respectif est déterminé, et dans lequel un résultat mesuré est obtenu sous forme d'une série d'au moins deux variables mesurées, qui est évaluée pour le diagnostic précis de la maladie neuro-dégénérative.

6. Procédé suivant la revendication 5, **caractérisé en ce que**, en tant qu'une partie de la détermination multiparamétrique, en plus de la détermination de mr-proANP, on détermine au moins un paramètre biochimique supplémentaire qui est choisi dans le groupe consistant en des médiateurs de l'inflammation, des constituants du complément, des cytokines, des chimiokines, des agents coagulants et facteurs fibrinolytiques du sang, des protéines de phase aiguë et des composés radicalaires.

7. Procédé suivant la revendication 5 ou 6, **caractérisé en ce que** la détermination multiparamétrique est effectuée sous forme d'une détermination simultanée au moyen d'un appareil de mesure utilisant la technologie des puces ou bien d'un dispositif de mesure immunochromatographique.

8. Procédé suivant l'une quelconque des revendications 5 à 7, **caractérisé en ce que** l'évaluation du résultat mesuré complexe de la détermination multiparamétrique est effectuée au moyen d'un programme informatique.
